# EUROPEAN PATENT APPLICATION

(11) **EP 4 079 359 A1**
(43) Date of publication of application: **26.10.2022**
(21) Application number: 21212395.4
(22) Date of filing: 03.12.2021
(51) Int. Cl.: A61M 16/06, A62B 18/00, F04B 19/00, A61M 16/00, A61M 16/16

(54) **HOSELESS CPAP MACHINE**

(30) Priority: 19.04.2021 US 202163176864 P; 29.10.2021 US 202117513978
(71) Applicant: Khan, Macksoud, Palo Alto, CA 94303 (US)
(72) Inventor: Khan, Macksoud, Palo Alto, CA 94303 (US)
(74) Representative: Sach, Greg Robert

(57) **Abstract**

Embodiments of the present invention may exist as a machine for continuous positive airway pressure, otherwise known as a "CPAP machine" in the art. The CPAP machine of the present invention may comprise a casing and plurality of working parts, the working parts including but not limited to a blower fan, one or more batteries, and a circuit board. All of the working parts may be housed within the casing, which allows the CPAP machine to exist as one compact unit that ban be easily worn by a user without use of a hose, tube, external wire, or other component used to connect a component of a CPAP machine to the portion of a CPAP machine that is worn on, over, or in front of a user's face.

## Description

This application claims priority of US Provisional Patent Application number 63/176,864 filed on April 19, 2021, and which is hereby incorporated by reference.

### Background of Invention

The present invention relates to systems and methods for providing continuous positive airway pressure (CPAP). Sleep apnea is a common health condition in which a person's breathing is slowed or paused during sleep. In common cases of sleep apnea, this irregular breathing results in snoring, which can be cumbersome for the afflicted person's partner. Sleep apnea can also result in severe health conditions such as high blood pressure or cardiac arrest.

To treat sleep apnea, CPAP machines provide a continuous flow of pressurized air towards a user's nose and/or mouth. The continuous, pressurized airflow creates a pneumatic cushion within a user's airway that prevents the user's airway from collapsing and prevents unwanted material such as mucus, etc. from clogging the user's airway.

CPAP machines create the continuous, pressurized airflow by use of an air compressor and/or blower fan. In most CPAP machines, air is taken from the ambient environment, pressurized in the compressor/blower fan, and pushed towards the user's nose and/or mouth. Most CPAP machines in the art comprise a hose that connects the air compressor/blower fan to a face mask. The face mask is then worn over the user's face in order to hold the hose near the user's nose and/or mouth.

The hose of most CPAP machines can be uncomfortable to users during sleep. This is a well-known problem in the art, such that special pillows and mask attachments have been developed to prevent CPAP machine hoses from interfering with CPAP machine users during sleep. However, these CPAP machine accessories can be expensive and impractical to travel with.

The presence of a hose in a CPAP machine signifies a compressor/blower fan that is detached from the facemask. This results in a bulky, non-portable CPAP machine that takes up an undesirably large amount of space in a user's bedroom. Said non-portable CPAP machines are also impractical for travel.

There exists a need in the art for a compact CPAP machine without a hose. This compact CPAP machine would provide the benefits of a common CPAP machine without the uncomfortable hose or expensive and cumbersome accessories. Furthermore, the compact nature of said CPAP machine would allow it to be easily carried with a user while traveling.

### Summary of Invention

Embodiments of the present invention may exist as a machine for continuous positive airway pressure, otherwise known as a "CPAP machine" in the art. The CPAP machine of the present invention may comprise a casing and plurality of working parts. The term "working parts" when used herein shall be defined as components of a basic battery-powered CPAP machine design that are integral for the machine to function as a CPAP machine. Said components may include but are not limited to a blower fan, one or more batteries, and a circuit board.

A blower fan is integral to any CPAP machine as it serves as the means for providing a continuous stream of air to the user of the CPAP machine. One or more batteries are integral to any battery-powered CPAP machine in order for a CPAP machine to be powered by batteries instead of a connection to a remote electrical source such as an electrical outlet. A circuit board is integral to any CPAP machine powered by electricity since the circuit board serves as a medium or substrate to house electrical components or electrical circuitry. For means of this description, a strap used to secure the CPAP machine to a user's face and/or head is not considered "integral for the machine to function as a CPAP machine." Though the working parts of some embodiments of the present invention are described thus far as a blower fan, one or more batteries, and a circuit board, other embodiments of the invention may comprise additional working parts that may comprise any other component described herein.

In some embodiments of the invention, the casing may be located in front of the user's face when the CPAP machine is in use. At least a portion of the casing may further rest against the user's face when the CPAP machine is in use. All of the plurality of working parts may be housed within the casing, which allows the CPAP machine to exist as one compact unit that can be easily worn by a user without use of a hose, tube, external wire, or other component used to connect a component of a CPAP machine to the portion of a CPAP machine worn on, over, or in front of the user's face. In embodiments of the present invention, the CPAP machine may be secured to the user's face and/or head by means of a strap.

Some embodiments of the invention further comprise a nasal pillow that may rest against the user's nostrils when the CPAP machine is in use. The nasal pillow may serve as an airway through which air may flow into the user's nostrils when the CPAP machine is in use. Some embodiments of the invention further comprise a cheek bumper that may rest against the user's cheek when the CPAP machine is in use. The nasal pillow and cheek bumper may be removably or permanently connected to the outside of the casing. In some embodiments of the invention, the nasal pillow and/or cheek bumper are the only components of the invention that rest against the user's face. In other embodiments of the invention, at least a portion of the casing also rests against the user's face. In still other embodiments, other components may also rest against the user's face in addition to the nasal pillow, cheek bumper, and/or casing. The nasal pillow, cheek bumper, and/or casing may be combined to form a face mask that fits on or over the user's face when the CPAP machine is in use. In the embodiments of the invention that do not comprise a nasal pillow or cheek bumper, the casing itself may serve as a face mask that fits on or over the user's face when the CPAP machine is in use.

In some embodiments of the invention, the CPAP machine may cover both the user's nose and mouth. In other embodiments, the CPAP machine may only cover the user's mouth, and comprise a nasal pillow that rests against the user's nostrils without covering the user's nose. In other embodiments, the CPAP machine may only cover the user's nose and not the user's mouth. In other embodiments, the CPAP machine may cover neither the user's nose nor mouth, or may cover only portions of the user's nose and/or mouth.

Some embodiments of the invention comprise a gyroscope, that may be one of the plurality of working parts. The gyroscope may be configured to send data to a user's device via a wireless network. The term "data" as used herein is defined as information in the form of non-transient, computer-readable media. The wireless network of some embodiments of the invention may be any wireless connection known in the art of wireless connections, including but not limited to a Bluetooth or WiFi connection. The term "user's device" as used herein is defined as any machine capable of sending and/or receiving data. The user's device may be a smartphone, tablet, general purpose computer, special purpose computer, or any other machine capable of sending and/or receiving information in the form of non-transient, computer-readable media.

In addition to sending data to the user's device, the gyroscope may further be configured to receive data from the user's device. The gyroscope may be configured to adjust certain parameters of the gyroscope and/or other working parts of the invention based on the data received from the user's device. Adjusting certain parameters of other working parts of the invention may comprise adjusting the speed of the blower fan.

In some embodiments of the invention, the circuit board may be configured to send data to and/or receive data from the user's device. In the embodiments of the invention where the circuit board is configured to receive data from the user's device, the circuit board may be configured to adjust certain parameters of the circuit board and/or other working parts of the invention based on the data received from the user's device. Adjusting certain parameters of other working parts of the invention may comprise adjusting the speed of the blower fan.

In some embodiments, the CPAP machine may comprise an air compressor in addition to the blower fan. In some of such embodiments, the air compressor may comprise a large diameter inlet tube, a fan, and a small diameter outlet tube. The large diameter inlet tube may be located anterior (further from the user's face) to the fan, which is located anterior to the small diameter outlet tube, which leads into the casing. In this manner, air may be drawn from the ambient environment into the large diameter inlet tube by the fan and may be compressed when entering the outlet tube that has a smaller diameter and thus a smaller volume. The term "tube" in the description of the air compressor herein refers to a manufactured airway and is not to be interpreted as a hose that exists in other CPAP machines in the art.

In some embodiments, the blower fan may be a piezo blower, which may use piezoelectric vibrations to generate air flow towards the user. The piezo blower may be powered by the one or more batteries and may be controlled by the circuit board. Some embodiments that comprise a piezo blower may also comprise an air compressor, though there may be embodiments that exist with solely the piezo blower and no air compressor.

Some embodiments of the invention may comprise a humidifier. The humidifier that exists in some embodiments of the invention may evaporate water and send said evaporated water into the air outputted by the blower fan and/or air compressor. The presence of humidified air in the CPAP machine may ease the introduction of continuous air into the user's airway and can be appreciated by those skilled in the art.

The structural components of the CPAP machine such as but not limited to the casing, cheek bumper, strap, and nasal pillow, may be designed to fit statistically common sizes of user's faces as they exist in various populations. In these embodiments and other embodiments, the structural components of the CPAP machine may have elastic properties and thus may fit securely over a user's face in order to minimize the loss of air flow from the space between the user's face and the casing. In other embodiments, the structural components of the CPAP machine may be available in various sizes, or may be custom-manufactured based on measurements of a user's face in order to create an optimal fit of the face mask to the user's face.

The components of the CPAP machine may be small enough and lightweight enough so that the entire invention may be comfortable worn by a user while sleeping. The size and weight of said components also allow the invention to fit inside of a travel bag when the invention is fully assembled.

### Brief Description of Figures

**Fig. 1** is a perspective view of a hoseless CPAP machine.
**Fig. 2** is an exploded view of a hoseless CPAP machine.
**Fig. 3A** is front view of a front portion of a casing for a hoseless CPAP machine.
**Fig. 3B** is right side view of a front portion of a casing for a hoseless CPAP machine.
**Fig. 3C** is back view of a front portion of a casing for a hoseless CPAP machine.
**Fig. 4A** is front view of a back portion of a casing for a hoseless CPAP machine.
**Fig. 4B** is right side view of a back portion of a casing for a hoseless CPAP machine.
**Fig. 4C** is back view of a back portion of a casing for a hoseless CPAP machine.
**Fig. 5A** is front view of a nasal pillow for a hoseless CPAP machine.
**Fig. 5B** is a bottom view of a nasal pillow for a hoseless CPAP machine.
**Fig. 6A** is a top view of a right cheek bumper for a hoseless CPAP machine.
**Fig. 6B** is a front view of a right cheek bumper for a hoseless CPAP machine.
**Fig. 7A** is a top view of a left cheek bumper for a hoseless CPAP machine.
**Fig. 7B** is a front view of a left cheek bumper for a hoseless CPAP machine.
**Fig. 8A** is a front view of a front rubber mount for a hoseless CPAP machine.
**Fig. 8B** is a left side view of a front rubber mount for a hoseless CPAP machine.
**Fig. 8C** is a back view of a front rubber mount for a hoseless CPAP machine.
**Fig. 9A** is a front view of a back rubber mount for a hoseless CPAP machine.
**Fig. 9B** is a left side view of a back rubber mount for a hoseless CPAP machine.
**Fig. 9C** is a back view of a back rubber mount for a hoseless CPAP machine.
**Fig. 10** is a front view of a strap for a hoseless CPAP machine.
**Fig. 11** is a bottom view of a lens for a hoseless CPAP machine.
**Fig. 12A** is front view of a rubber block for a hoseless CPAP machine.
**Fig. 12B** is side view of a rubber block for a hoseless CPAP machine.

### Detailed Description

The description provided herein describes example embodiments of the invention and is not intended to limit the invention to any particular embodiments or features. Likewise, the figures provided herein are for purpose of example, and are not intended to limit the invention to any particular features, size, shape, color, or any other functional or aesthetic property. It shall be noted that specific dimensions and dimensional ranges are mentioned throughout this description to describe the possible sizes of various components of the invention. Said dimensions and dimensional ranges describe example embodiments and are not necessarily indicative of the size of each and every possible embodiment of the invention. Dimensional ranges listed herein are intended to be inclusive. For example, a range stated as "4mm to 9mm" is intended to comprise both 4mm and 9mm.

The various dimensions of the components of the present invention mentioned herein are used to show that the present invention and its components may exist in a variety of shapes and sizes. Components with smaller dimensions may be applicable for a CPAP machine used by a user with smaller anatomical dimensions, whereas components with larger dimensions may be applicable for a CPAP machine used by a user with larger anatomical dimensions. Components may further be dimensioned appropriately to be used with CPAP machines that are shaped differently than shown in the figures provided herein.

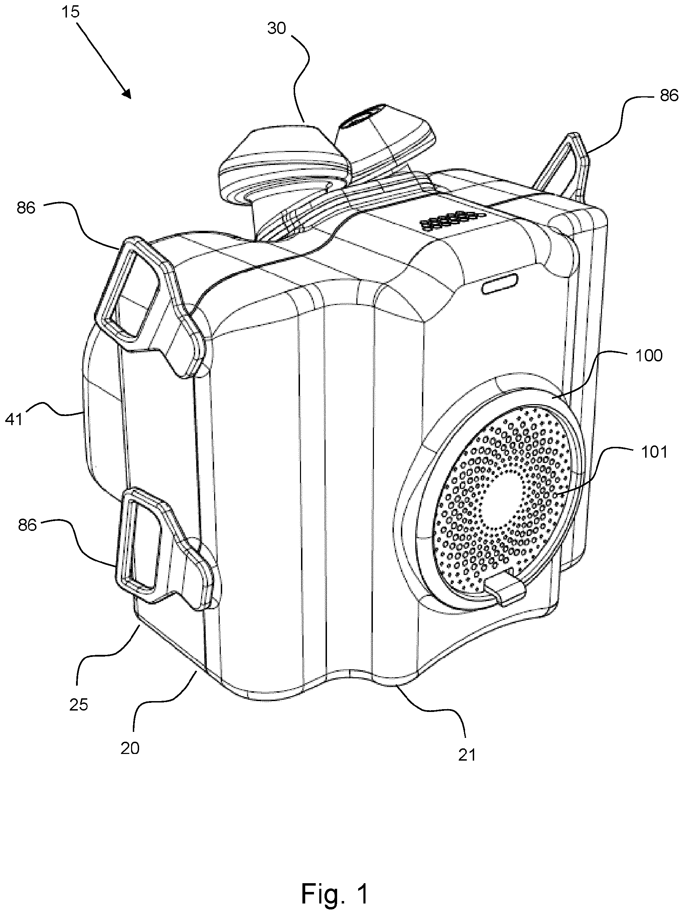

**Fig. 1** shows a hoseless CPAP machine **15,** which comprises a casing **20,** the casing **20** comprising a front portion **21** and a back portion **25.** The front portion **21** of the casing **20** comprises a hole which is covered by an air grill **101.** The hole covered by the air grill **101** may serve as the orifice through which ambient air enters the hoseless CPAP machine **15.** A bevel **100** is situated over the air grill **101** and covers the edges of the air grill **101** that are in contact with front portion **21** of the casing **20.** Connected to the casing **20** are a plurality of strap clips **86.** Three strap clips **86** are show in the perspective view that is **Fig. 1****,** though the invention may comprise one or more strap clips **86.** Also connected to the casing **20** is a nasal pillow **30.** The nasal pillow **30** may rest against or be partially inserted into a user's nostrils when the hoseless CPAP machine **15** is in use. Air may flow from the hoseless CPAP machine **15,** through the nasal pillow **30,** and into the user's nostrils when the hoseless CPAP machine **15** is in use. Also connected to the casing **20** as shown in **Fig. 1** is a right cheek bumper **41,** that may rest against a user's cheek when the hoseless CPAP machine **10** is in use.

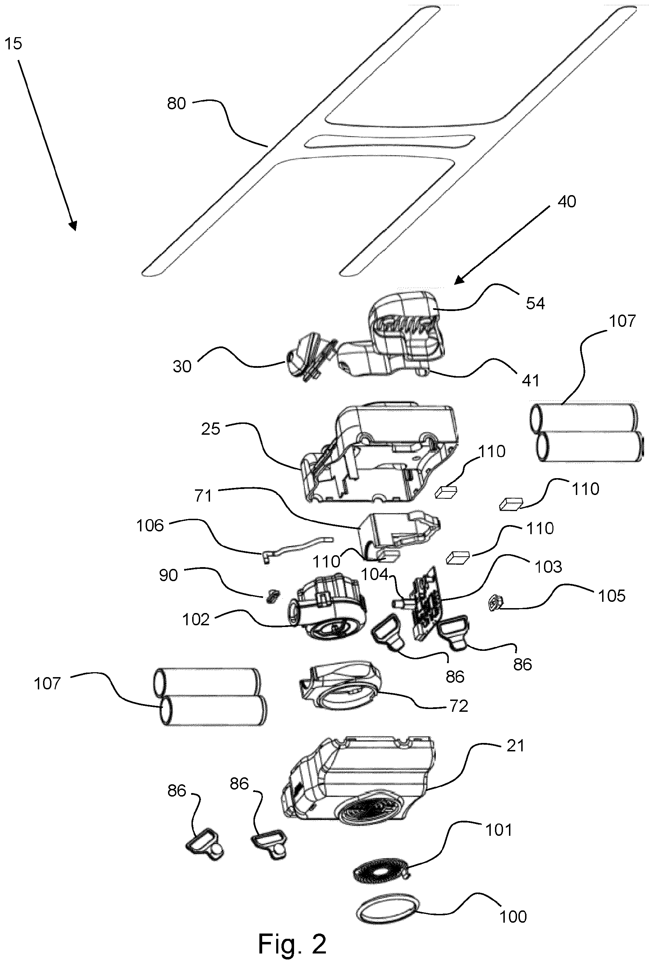

**Fig. 2** shows an exploded view of the hoseless CPAP machine **15** from **Fig. 1****.** The casing front portion **21,** casing back portion **25,** bevel **100,** air grill **101,** nasal pillow **30,** right cheek bumper **41,** and strap clips **86** from **Fig. 1** are all shown in **Fig. 2****.** Because of the view of **Fig. 2****,** four strap clips **86** are shown. A strap **80** is also shown in **Fig. 2****.** The strap clips **86** are removably attached to the casing **20** and serve to hold the strap **80** to the hoseless CPAP machine **15.** The casing **20** houses a front rubber mount **72** and back rubber mount **71,** that may be attached to form one rubber mount unit, also referred to herein simply as a "rubber mount." The rubber mount houses a blower fan **102** which pulls ambient air from the environment, through the hoseless CPAP machine **15,** and into one or more orifices of a user when the hoseless CPAP machine **15** is worn by a user. The rubber mount may serve to provide a means of attaching the blower fan **102** inside of the casing **20,** as well as to provide a means for absorbing vibrations caused by the blower fan **102** so that said vibrations are not transferred to the casing 20 or other components of the hoseless CPAP machine **15.**

Also shown in **Fig. 2** are two batteries **107.** The batteries are housed within the casing **20** and serve to power the blower fan **102** and other optional components of the hoseless CPAP machine **15** such as a gyroscope. The batteries **107** are electrically coupled to a circuit board **103.** A power button **105** is also electrically coupled to the circuit board **103** and serves as a switch for the circuit so that a user may power the hoseless CPAP machine **15** on and off. The various other powered components of the invention such as the blower fan **102,** gyroscope, humidifier, dehumidifier, and other sensors may also be electrically coupled to the circuit board **103.** The batteries **107** are cushioned by a plurality of rubber blocks **110** that may be made of natural rubber or other rubber. Rubber may be used to cushion the batteries **107** rather than foam since foam can degrade over time. Degradation of components used to cushion batteries is an issue with other CPAP machines that exist in the art.

The circuit board **103** comprises an LED (not shown) that may display a plurality of colors as well as perform a plurality of blinking patterns. Said colors and blinking patterns may be used to communicate the state of the CPAP machine **15** to the user. A light tube adapter **104** is fitted over the LED, and one end of a light tube **106** is connected to the light tube adapter **104.** Another end of the light tube **106** is connected to a lens **90** which is connected to the outside of the casing **20.** This connection between the LED, light tube adapter **104,** light tube **206,** and lens **90** allows light from the LED to shine outside of the casing **20** in order to be viewed by the user, even when the circuit board **103** where the LED is located is within the casing **20** and away from the edge of the casing **20.**

Not shown in **Fig. 2** are the gyroscope and other optional powered components of the invention. The other optional powered components may include but are not limited to a thermometer to measure air temperature, a barometer to measure air pressure, a humidifier to increase the humidity of the air being inhaled by the user when the invention is in use, a dehumidifier to decrease the humidity of the air being inhaled by the user when the invention is in use, and a transmitter used to transmit wireless signals from the circuit board **103** to a user's device, such as a smartphone, laptop, or tablet.

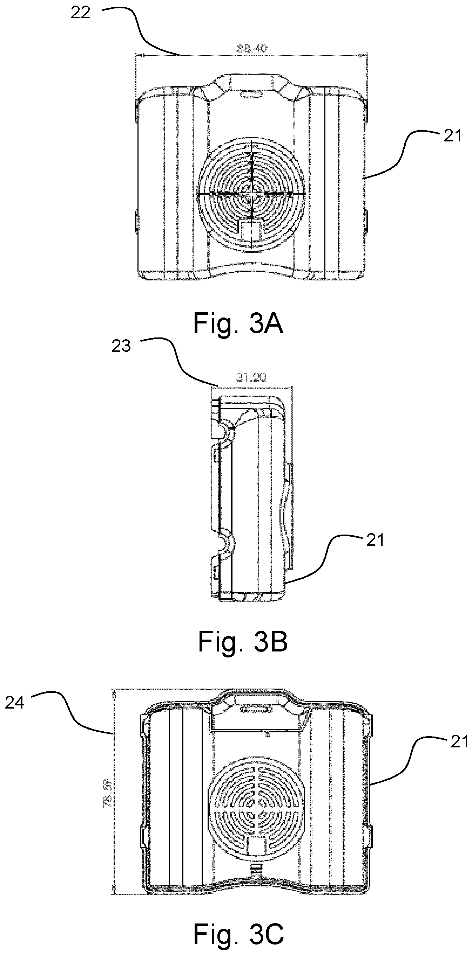

**Fig. 3A** shows a front view of the front portion **21** of the casing without the air grill or bevel attached. The front portion **21** of the casing is shown with an overall length **22** of 88.04mm. In other embodiments of the invention, this overall length **22** may be anywhere within the range of 60mm to 120mm. **Fig. 3B** shows a right side view of the front portion **21** of the casing without the air grill or bevel attached. The front portion **21** of the casing is shown with an overall width **23** of 31.20mm. In other embodiments of the invention, this overall width **23** may be anywhere within the range of 10mm to 50mm. **Fig. 3C** shows a back view of the front portion **21** of the casing without the air grill or bevel attached. The front portion **21** of the casing is shown with an overall height **24** of 78.59mm. In other embodiments of the invention, this overall height **24** may be anywhere within the range of 60mm to 120mm.

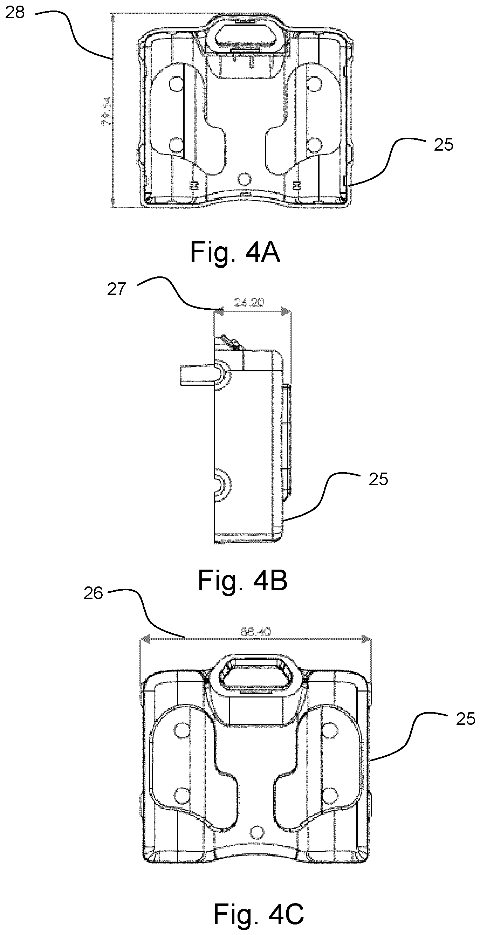

**Fig. 4A** shows a front view of the back portion **25** of the casing. The back portion **25** of the casing is shown with an overall height **28** of 79.54mm. In other embodiments of the invention, this overall height **28** may be anywhere within the range of 60mm to 120mm. **Fig. 4B** shows a right side view of the back portion **25** of the casing. The back portion **25** of the casing is shown with an overall width **27** of 26.20mm. In other embodiments of the invention, this overall width **27** may be anywhere within the range of 10mm to 50mm. **Fig. 4C** shows a back view of the back portion **25** of the casing without the air grill or bevel attached. The back portion **25** of the casing is shown with an overall length **26** of 88.40mm. In other embodiments of the invention, this overall length **26** may be anywhere within the range of 60mm to 120mm.

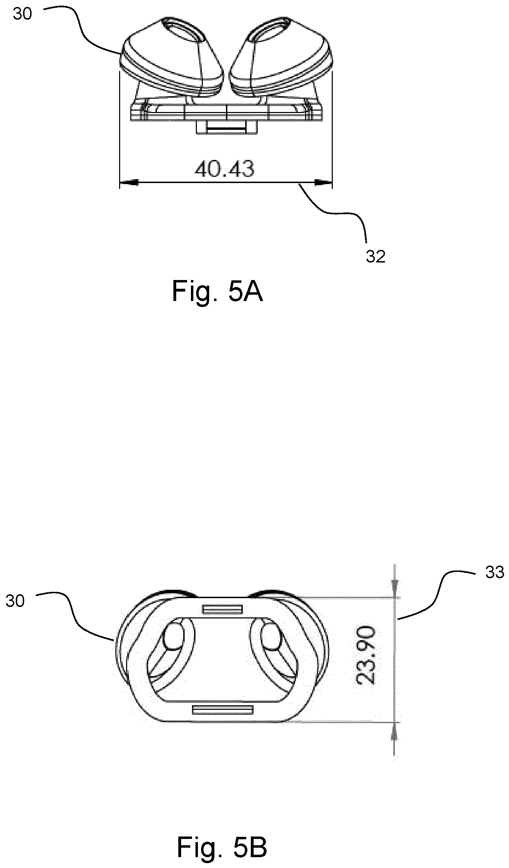

**Fig. 5A** shows a front view of the nasal pillow **30** with an overall length **32** of 40.43mm. In other embodiments of the invention, this overall length **32** may be anywhere within the range of 10mm to 60mm. **Fig. 5B** shows a bottom view of the nasal pillow **30** with an overall width **33** of 23.90mm. In other embodiments of the invention, this overall width **33** may be anywhere in the range of 10mm to 40mm. The nasal pillow **30** may also come in various sizes as to allow the hoseless CPAP machine to be better adapted to different users' anatomies. In some embodiments, the nasal pillow **30** may come in a small size, a medium size that is larger than the small size, and a large size that is larger than the medium size.

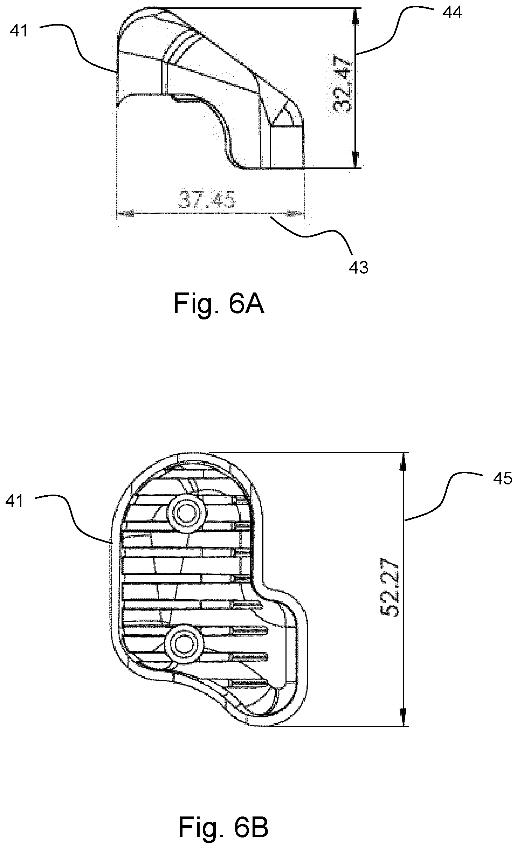

**Fig. 6A** shows a top view of the right cheek bumper **41** with an overall width **44** of 32.47mm and an overall length **43** of 37.45mm. In other embodiments of the invention, this overall width **44** and overall length **43** may each be anywhere within the range of 15mm to 50mm. **Fig. 6B** shows a front view of the right cheek bumper **41** with an overall height **45** of 52.27mm. In other embodiments of the invention, this overall height **45** may be anywhere in the range of 30mm to 70mm. The right cheek bumper **41** may also come in various sizes to allow the hoseless CPAP machine to be adaptable to different users' anatomies. In some embodiments, the right cheek bumper **41** may come in a small size, a medium size that is larger than the small size, and a large size that is larger than the medium size.

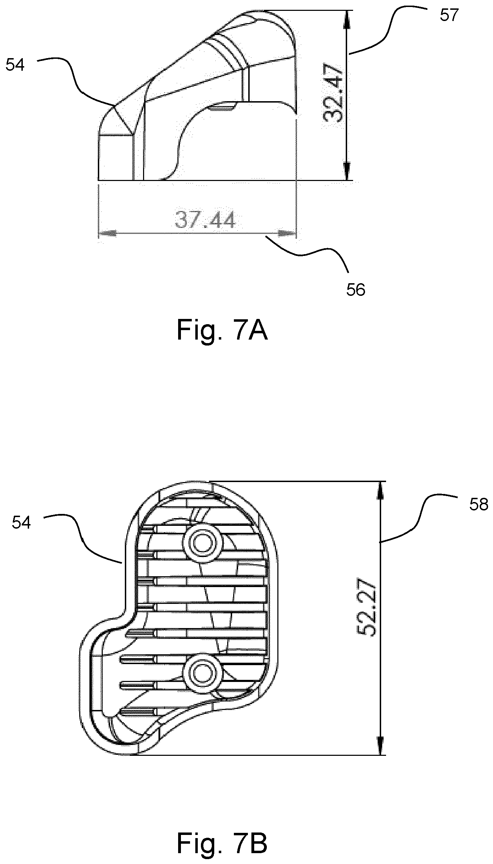

**Fig. 7A** shows a top view of the left cheek bumper **54** with an overall width **57** of 32.47mm and an overall length **56** of 37.44mm. In other embodiments of the invention, this overall width **57** and overall length **56** may each be anywhere within the range of 15mm to 50mm. **Fig. 7B** shows a front view of the left cheek bumper **54** with an overall height **58** of 52.27mm. In other embodiments of the invention, this overall height **58** may be anywhere in the range of 30mm to 70mm. The left cheek bumper **54** may also come in various sizes to allow the hoseless CPAP machine to be adaptable to different users' anatomies. In some embodiments, the left cheek bumper **54** may come in a small size, a medium size that is larger than the small size, and a large size that is larger than the medium size.

The right cheek bumper and left cheek bumper may be removably or permanently attached to form one cheek bumper unit, also referred to herein as simply a "cheek bumper." The cheek bumper and components thereof may come in a plurality of sizes and/or configurations to allow the hoseless CPAP machine to be adaptable to different users' anatomies. The right cheek bumper may rest against the user's right cheek when the invention is in use, and the left cheek bumper may rest against a user's left cheek when the invention is in use. The cheek bumper may create a seal against the user's cheeks so that air that passes through the hoseless CPAP machine does not escape around the side of the user's face.

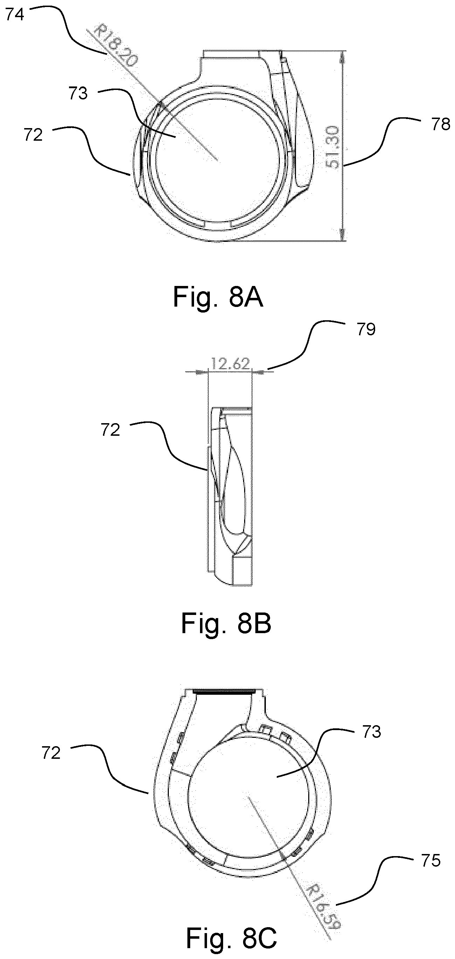

**Fig. 8A** shows a front view of the front rubber mount **72,** the front rubber mount comprising a hole **73.** The hole **73** extends through the entire width of the front rubber mount **72** and may serve as a passageway through which air may travel from the environment and into the blower fan. The hole **73** is shown in **Fig. 8A** with a front radius **74** of 18.20mm. In other embodiments of the invention, this front radius **74** may be anywhere in the range of 10mm to 30mm. The front rubber mount **72** is shown in **Fig. 8A** with an overall height **78** of 51.30mm. In other embodiments of the invention, this overall height **51.30** may be anywhere in the range of 30mm to 70mm. **Fig. 8B** shows a left side view of the front rubber mount **72** with an overall width 79 of 12.62mm. In other embodiments of the invention, this overall width **79** may be anywhere within the range of 5mm to 25mm.

**Fig. 8C** shows a back view of the front rubber mount **72.** **Fig. 8C** also shown the hole **73** with a back radius **75** of 16.59mm. In other embodiments of the invention, this back radius **75** may be anywhere in the range of 10mm to 30mm. When the front radius **74** is greater than the back radius **75,** the front rubber mount **72** acts as a nozzle to increase the pressure of the air going through the front rubber mount **72** and to decrease the temperature of the air going through the front rubber mount **72.** When the front radius **74** is less than the back radius **75,** the front rubber mount **72** acts as a diffuser to decrease the pressure of the air going through the front rubber mount **72** and increase the temperature of the air going through the front rubber mount **72.** Either may be preferable depending on the specific needs and preferences of the user.

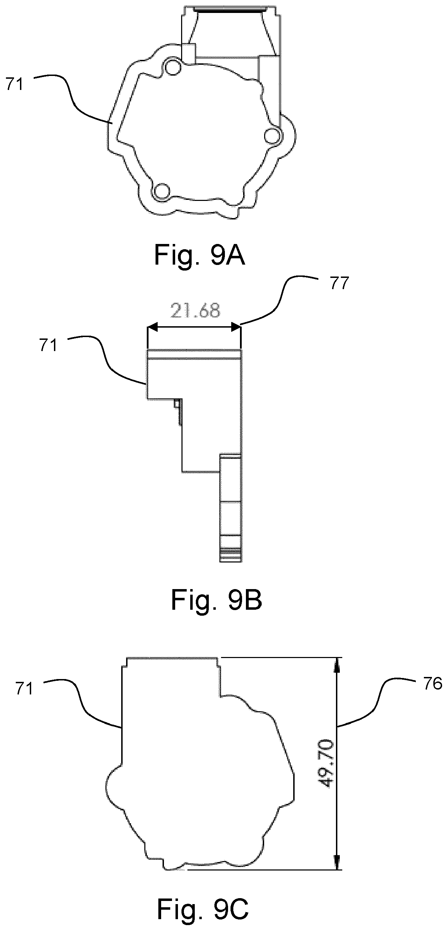

**Fig. 9A** shows a front view of the back rubber mount **71** that may be attached to the front rubber mount **72** in order to create the rubber mount, which may be used to encase the blower fan **102.** **Fig. 9B** shows a left side view of the back rubber mount **71** with an overall width **77** of 21.68mm. In other embodiments of the invention, this overall width **77** may be anywhere in the range of 10mm to 30mm. **Fig. 9C** shows a back view of the back rubber mount **71** with an overall height **76** of 49.70mm. In other embodiments of the invention, this overall height **76** may be anywhere in the range of 30mm to 70mm. The back rubber mount **71** in **Figs. 9A** - **9B** is not shown with a hole similar to the hole **73** of the front rubber mount **72.** In these embodiments, air is moved from the blower fan **102** to the user's nostrils via the nasal pillow **30.** In other embodiments of the invention, the back rubber mount **71** may comprise a hole so that air may flow from the blower fan to the user's mouth.

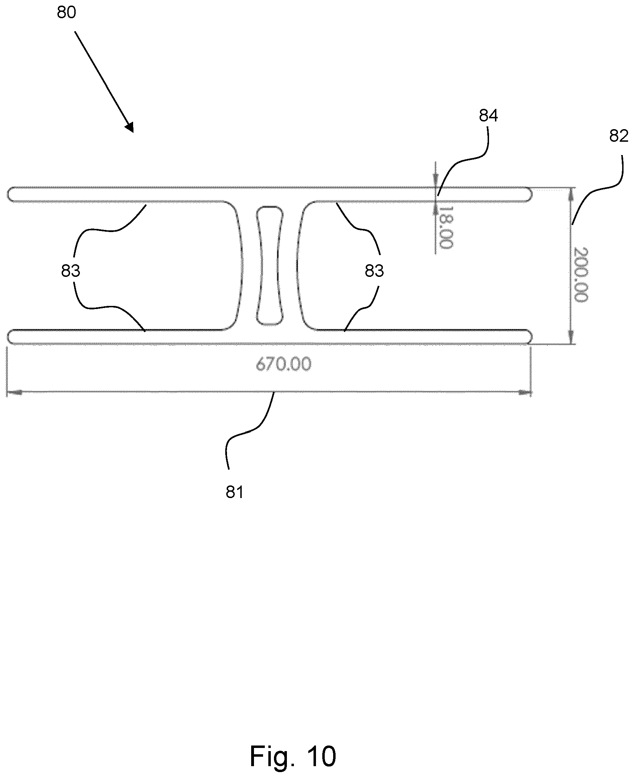

**Fig. 10** shows a front view of the strap **80,** which comprises 4 arms **83.** The arms of the strap may be fitted through the strap clips **86** in order to secure the strap to the hoseless CPAP machine **15.** The rest of the strap is then placed around the back of the user's head in order to secure the hoseless CPAP machine **15** to the user's face. The strap **80** in **Fig. 10** is shown with an overall length **81** of 670mm. In other embodiments of the invention, this overall length **81** may be anywhere within the range of 500mm to 750mm. Furthermore, the strap **80** may be made of an elastic material such as rubber or silicone so that the strap **80** may stretch to an overall length **81** greater than 750mm. The strap **80** in **Fig. 10** is shown with an overall width **82** of 200mm. In other embodiments of the invention, this overall width **82** may be anywhere within the range of 50mm to 350mm. Furthermore, the elastic properties of some embodiments of the strap **80** may allow the strap **80** to stretch to an overall width **82** greater than 350mm. The arms **83** of the strap **80** are shown in **Fig. 10** with thicknesses **84** of 18mm. In other embodiments of the invention, the thicknesses **84** of the arms **83** may be anywhere within the range of 10mm to 30mm. Furthermore, the elastic properties of some embodiments of the strap **80** may allow the arms **83** to expand to overall thicknesses **84** greater than 30mm.

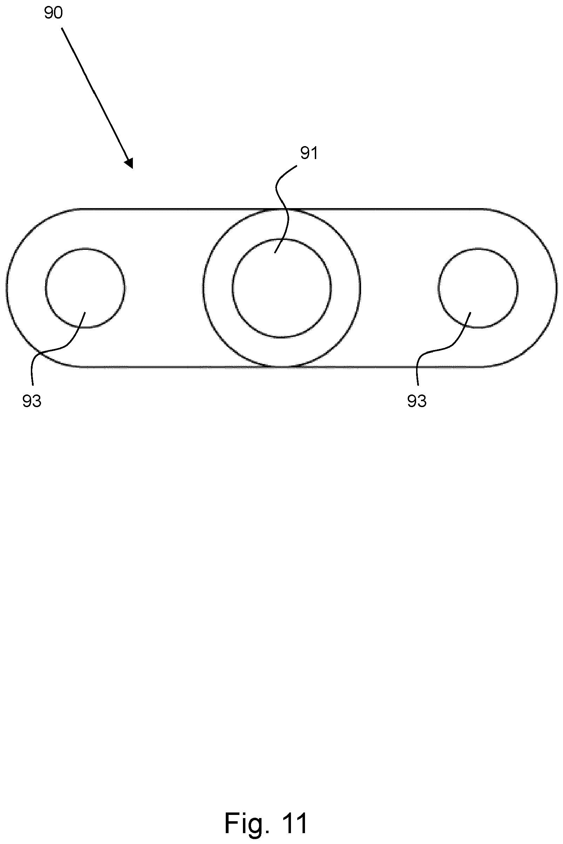

**Fig. 11** shows a bottom view of the lens **90** with a center hole **91** and 2 peripheral holes **93.** The peripheral holes **93** are used to attach the lens **90** to the casing **20.** The center hole **91** is used to accept the light tube **106** so that light from the LED of the circuit board **103** can be seen from outside of the casing **20.**

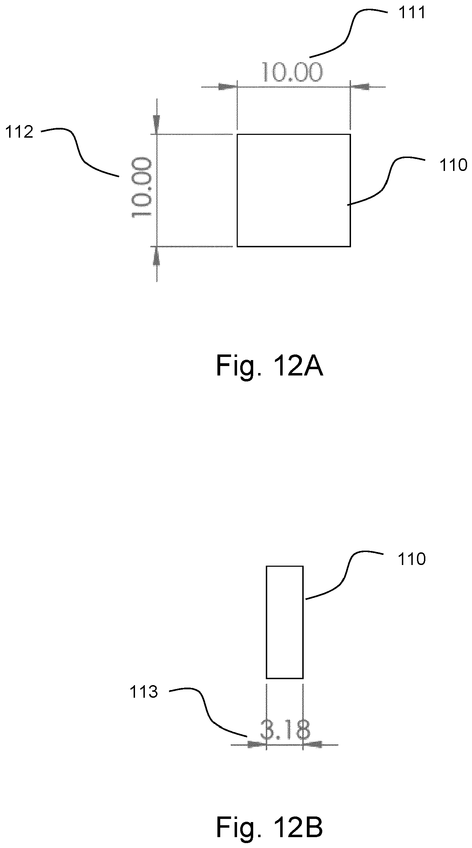

**Fig. 12A** shows a front view of one of the rubber blocks **110** with an overall length **111** of 10mm and an overall width **112** of 10mm. In other embodiments of the invention, this overall length **111** and overall width **112** may each be anywhere within the range of 5mm to 15mm. Furthermore, this overall length **111** and overall width **112** may be equal in some embodiments of the invention, but are not always equal in all embodiments of the invention. **Fig. 12B** shows the rubber block **110** with a thickness **113** of 3.18mm. In other embodiments of the invention, this thickness **113** may be anywhere within the range of 0.5mm to 5mm.

## Claims

1. A CPAP machine comprising:
a casing; and
a plurality of working parts, said working parts comprising a blower fan, one or more batteries, and a circuit board,
wherein the CPAP machine is worn over a user's nose and/or mouth so that the casing is located in front of the user's face when the CPAP machine is in use, and wherein all of the plurality of working parts are housed within the casing.

2. The CPAP machine of **Claim 1,** further comprising a nasal pillow that rests against the user's nostrils and serves as an airway through which air flows into the user's nostrils when the CPAP machine is in use.

3. The CPAP machine of **Claim 1,** further comprising a cheek bumper that rests against the user's cheeks when the CPAP machine is in use.

4. The CPAP machine of **Claim 1,** further comprising a strap that secures the CPAP machine to the user's head.

5. The CPAP machine of **Claim 1,** wherein the plurality of working parts further comprises a gyroscope.

6. The CPAP machine of **Claim 5,** wherein the gyroscope is configured to send data to a user's device via a wireless connection.

7. The CPAP machine of **Claim 1,** wherein the one or more batteries are rechargeable.

8. The CPAP machine of **Claim 1,** wherein the circuit board is configured to send data to a user's device via a wireless connection.

9. The CPAP machine of **Claim 8,** wherein the circuit board is configured to receive data sent from the user's device via the wireless connection.

10. A CPAP machine comprising:
a casing;
a strap; and
a plurality of working parts, said working parts comprising a blower fan, one or more batteries, and a circuit board,
wherein when the CPAP machine is in use, the casing rests against a user's face and is secured to the user's head by means of the strap, and wherein when the CPAP machine rests against the user's face the CPAP machine covers the user's nose and mouth, and
wherein all of the plurality of working parts are housed within the casing.

11. The CPAP machine of **Claim 10,** further comprising a nasal pillow that rests against the user's nostrils and serves as an airway through which air flows into a user's nostrils when the CPAP machine is in use.

12. The CPAP machine of **Claim 10,** further comprising a cheek bumper that rests against the user's cheeks when the CPAP machine is in use.

13. The CPAP machine of **Claim 10,** wherein the plurality of working parts further comprises a gyroscope, optionally
wherein the gyroscope is configured to send data to a user's device via a wireless connection.

14. The CPAP machine of **Claim 10,** wherein the one or more batteries are rechargeable.

15. The CPAP machine of **Claim 10,** wherein the circuit board is configured to send data to a user's device via a wireless connection, optionally
wherein the circuit board is configured to receive data sent from the user's device via the wireless connection.
